# EUROPÄISCHE PATENTSCHRIFT

(11) **EP 3 874 961 B1**
(45) Veröffentlichungstag und Bekanntmachung des Hinweises auf die Patenterteilung: **03.07.2024**
(21) Anmeldenummer: 21158986.6
(22) Anmeldetag: 24.02.2021
(51) Int. Cl.: A23F 5/10, A23F 5/16, G01N 33/02

(54) **AUSGASUNGSANORDNUNG FÜR FRISCH GERÖSTETEN KAFFEE SOWIE VERFAHREN ZUM LAGERN VON FRISCH GERÖSTETEM KAFFEE**
OUTGASING ASSEMBLY FOR FRESH ROASTED COFFEE AND METHOD FOR STORING FRESHLY ROASTED COFFEE
SYSTÈME DE DÉGAZAGE DU CAFÉ FRAÎCHEMENT TORRÉFIÉ, AINSI QUE PROCÉDÉ DE STOCKAGE DU CAFÉ FRAÎCHEMENT TORRÉFIÉ

(30) Priorität: 03.03.2020 DE 102020105699
(43) Veröffentlichungstag der Anmeldung: 08.09.2021
(73) Patentinhaber: PROBAT SE, 46446 Emmerich (DE)
(72) Erfinder: Koziorowski, Thomas, 46446 Emmerich (DE); Elshoff, Thomas, 46446 Emmerich (DE)
(74) Vertreter: terpatent Patentanwälte und European Patent Attorneys ter Smitten Eberlein -Van Hoof

(56) Entgegenhaltungen:
- EP-A2- 0 678 244
- WO-A1-2015/074149
- WO-A1-2019/106446
- GB-A- 2 128 068

## Beschreibung

Die Erfindung betrifft eine Ausgasungsanordnung für frisch gerösteten Kaffee mit einem Behälterorgan, in dem der Kaffee gelagert ist, dass zumindest eine Einlass- bzw. eine Auslassöffnung aufweist, wobei zumindest eine CO₂-Sensoranordnung mit zumindest einer Ansaugpumpe, einem CO₂-Sensororgan und einer Auswerteanordnung für den CO₂-Gehalt eines dem Behälterorgan entnommenen Analysegases zur Überwachung eines Ausgasungsprozesses vorgesehen ist. Die Erfindung betrifft ebenfalls ein Verfahren zum Lagern von frisch geröstetem Kaffee in einer derartigen Ausgasungsanordnung, wobei in der Auswerteanordnung ein CO₂-Endwert für den CO₂-Gehalt hinterlegt ist, mit dem der jeweilige aktuell gemessene CO₂-Gehalt vergleichbar ist, derart, dass bei Erreichen des CO₂-Endwertes der Ausgasungsprozess zu beenden ist, die über eine Absaugleitung mit dem Behälterorgan fluidisch verbunden ist.

Es ist beispielsweise aus der EP 0 678 244 A2 oder auch der WO 2019/106446 A1 bekannt, frisch gerösteten Kaffee in bohnenform oder auch gemahlen für einen bestimmten Zeitraum zu lagern, bevor der Kaffee in geeigneter Form verpackt wird. Diese "Lagerung" dient der Ausgasung eines Gasgemisches, wobei das CO₂-Gas als Leitgröße dient, welches Gasgenmisch während des Röstprozesses entstanden ist, wodurch die Kaffeezellen unter einem gewissen Druck stehen. Je nach Röstverfahren, Kaffeesorte, Kaffeezustand (gemahlen oder in Bohnenform) und bei gemahlenem Zustand dem Mahlgrad, ist die Gasentwicklung im Kaffee unterschiedlich ausgeprägt, so dass auch der Ausgasungsvorgang unterschiedlich ausgeprägt ist. Ein zu hoher Restgehalt an CO₂ im Kaffee könnte ein Bersten der jeweiligen Verpackungsart, beispielsweise Tüte, Dose oder auch Kapsel, bewirken. Um die Ausgangszeit zu verkürzen, ist es bekannt, die Ausgasung in einem unter Vakuum stehenden Behälterorgan ablaufen zu lassen. Es ist auch bekannt oder auch bei Überschreitung eines gewissen O₂-Gehaltes ein Inertgas dem Behälterorgan zuzuführen und CO₂-Gas abzuführen. Eine derartige Anordnung bzw. ein derartiges Verfahren ist in der US-amerikanischen Patentschrift US 10,314,319 B2 bzw. der WO 2015/074149 A1 beschrieben. Hierdurch soll gewährleistet werden, dass der Ausgasungsprozess beschleunigt stattfinden kann und demzufolge nach Ablauf einer möglichst kurzen Zeitspanne der Kaffee der weiteren Verpackung zugeführt werden kann. Es hat sich jedoch nun herausgestellt, dass dieser Erfahrungswert zur Ausgasungsdauer zu wesentlich größeren Unterschieden bei der Kaffeequalität des zu verpackenden Kaffees führt als bisher angenommen. Gründe hierfür können unterschiedliche Umgebungsfaktoren, wie zum Beispiel Temperatur oder auch Feuchtegehalt sein. Zudem ist das Ermitteln dieser Erfahrungswerte für die unterschiedlichen Kaffeezustände sehr aufwendig und damit teuer. Eine ähnliche Ausgasungsanordnung offenbart die britische Offenlegungsschrift GB 2 128 068 A, wobei die CO₂-Sensoranordnung über eine Absaugleitung mit der Auslassöffnung verbunden ist. Auch diese Anordnung liefert nicht die heute gewünschte Genauigkeit hinsichtlich der Ausgasung des zu verpackenden Kaffees.

Aufgabe der vorliegenden Erfindung ist es daher, die oben genannten Nachteile auf einfache und kostengünstige Weise zu vermeiden.

Diese Aufgabe wird dadurch gelöst, dass die Sensoranordnung eine Analyselanze aufweist, die in das Behälterorgan hineinreicht und die fluidisch über die Absaugleitung mit einer außerhalb des Behälterorgans angeordneten Transmitterbox, die zumindest eine Ansaugpumpe und ein CO₂-Sensororgan aufweist, verbunden ist, wobei die Transmitterbox kabellos oder kabelgebunden mit einer Steuereinrichtung verbunden ist, wobei die Analyselanze in einem unteren oder mittleren Bereich des Behälterorgans vorgesehen ist, derart, dass die Analyselanze in den Kaffee hineinreicht. Durch eine derartige Ausgasungsanordnung ist es möglich, den Ausgasungsprozess individuell, abgestimmt auf den Kundenwunsch, zu beenden. Auch sind exakt wiederholbare Kaffeezustände, zumindest hinsichtlich des CO₂-Gehaltes, möglich. Durch die Analyselanze ist es möglich, dass Analysegas zentral aus dem Behälterorgan abzusaugen, wodurch wesentlich genauere Messwerte ermittelt werden können. Anstatt einer Analyselanze ist es auch möglich andere Hilfsmittel bei der Sensoranordnung einzusetzen. So kann auch ein halboffenes Rohr als Absaugmittel dienen. Wichtig ist auf jeden Fall, dass die Absaugung zentral im Behälterorgan stattfindet. Durch eine derartige Messung in der Kaffeesäule kann eine hohe Konzentration (in Volumen-%) des CO₂-Gehaltes erfasst werden. Äußere Einflüsse werden auf diese Weise so gut wie vollständig vermieden.

Um die Randbedingung für die Ausgasung durch das entnommene Analysegas nicht zu ändern, kann es vorteilhaft sein, eine Rückführleitung zur Rückführung des Analysegases von der Transmitterbox in das Behälterorgan vorzusehen. In besonders vorteilhafter Weise ist in der Transmitterbox ein O₂-Sensororgan zur Messung des O₂-Gehaltes vorgesehen. In vorteilhafter Weise ist ein Ablassorgan, insbesondere ein Ventil, am Behälterorgan vorgesehen, um Gas abzulassen. Um den O₂-Gehalt im Behälterorgan möglichst konstant zu halten und damit eine Oxidation des Kaffees weitest möglich zu vermeiden, kann es insbesondere vorteilhaft sein, ein Inertgas-Zuführorgan am Behälterorgan vorzusehen, um Inertgas dem Behälterorgan zuzuführen. In besonders vorteilhafter Weise kann hierbei das Inertgas-Zuführorgan durch die Analyselanze ausgebildet sein.

Eine besonders komfortable Ausgasungsanordnung wird dadurch bereitgestellt, dass in der Auswerteanordnung unterschiedliche CO₂-ZeitDiagramme für unterschiedliche Kaffeezustände, wie zum Beispiel unterschiedliche Kaffeesorten, Röstgrade, Röstzeiten, Mahlgrad, etc. hinterlegt sind. Eine derartige Ausgasungsanordnung ist insbesondere für eine automatisierte Kaffeebearbeitung geeignet, bei der das jeweilige Behälterorgan durch eine Steuereinheit automatisch entleert wird. Die Auswerteanordnung ist dann auf bekannte Weise steuerungstechnisch mit der Steuereinheit zu verbinden.

Die Aufgabe wird ebenfalls durch ein Verfahren zum Lagern von frisch geröstetem Kaffee, das heißt in Form von Kaffeebohnen oder Mahlkaffee, in einer derartigen Ausgasungsanordnung gelöst, wobei Kaffee in das Behälterorgan eingefüllt wird, der Ausgasungsprozess mittels der Auswerteanordnung überwacht wird und wobei der Ausgasungsprozess und damit ein Lagerprozess beendet wird, sobald ein vordefinierter CO₂-Endwert in einem vordefinierten (Zeit)-bereich erreicht wird. Hierbei kann der (Zeit)-bereich mit der maximal möglichen Dauer des Lagerprozesses übereinstimmen. Im Falle einer stark ansteigenden CO₂-Ausgasung und einem nachfolgenden Abfall des CO₂-Gehaltes ist der Zeitbereich jedoch im Bereich des Abfalles des CO₂-Gehaltes zu wählen, um sicher zu stellen, dass der Lagerprozess nicht zu früh (bei Ansteigen des CO₂-Gehaltes) beendet wird.

Die Erfindung wird anhand einer Zeichnung näher erläutert, hierbei zeigt:
Figur 1 eine schematische Ansicht einer erfindungsgemäß Ausgasungsanordnung,
Figur 2 ein Diagramm, bei dem ein CO₂- bzw. O₂-Gehalt gegenüber der Zeit von gemahlenem Filterkaffee und gemahlenem Espressokaffee in einer Ausgasungsanordnung mit einem nicht-gasdichten Behälterorgan dargestellt ist, und
Figur 3 ein CO₂- /O₂-Gehalt-Zeitdiagramm bei dem Columbia-Arabica-Kaffeebohnen mit Vietnam-Robusta-Kaffeebohnen, gelagert in einem gasdichten Behälterorgan, verglichen werden.

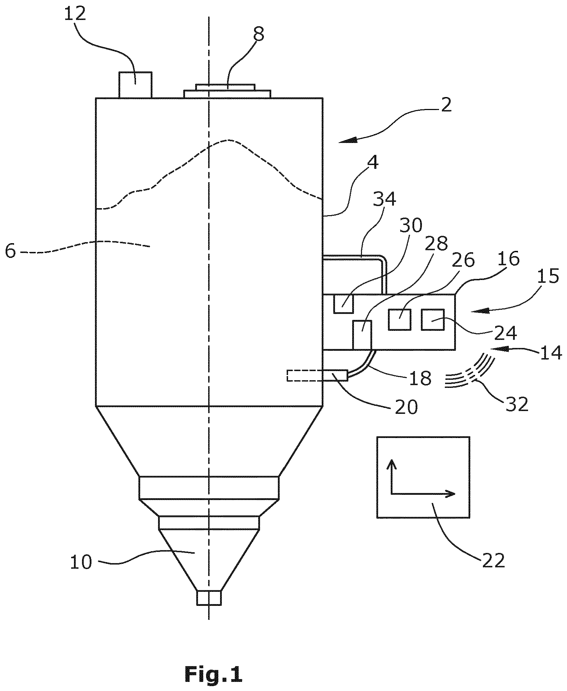

Figur 1 zeigt eine schematische Ansicht einer erfindungsgemäßen Ausgasungsanordnung 2. Diese Ausgasungsanordnung 2 weist ein Behälterorgan 4 auf, das im vorliegenden Ausführungsbeispiel mit frisch geröstetem und gemahlenem Mahlkaffee 6 befüllt ist. Im vorliegenden Ausführungsbeispiel erfolgte die Befüllung mit dem Mahlkaffee 6 zu 3/4 des Volumens des Behälterorgans 4, um Behältervolumen für einen Ausgasungsprozess des Mahlkaffees vorzuhalten. Auf bekannte Weise besitzt das Behälterorgan 4 eine Einlassöffnung 8, durch die der auszugasende Kaffee 6 in das Behälterorgan 4 gefüllt wird und eine Auslassöffnung 10, durch die eine Entleerung des Behälterorgans 4 stattfindet. Es sollte deutlich sein, dass Einlass- und Auslassöffnungen 8, 10 auch übereinstimmen können.

Die vorliegende Ausgasungsanordnung 2 besitzt im vorliegenden Ausführungsbeispiel ein Ablassorgan 12, durch das bei einem zu hohen Druck Gas, im wesentlichen CO₂, aus dem Behälterorgan 4 abgelassen wird. Im vorliegenden Ausführungsbeispiel weist die Ausgasungsanordnung 2 ein bis zu einem bestimmten Druck dichtes Behälterorgan 4 auf. Ein Druckausgleich kann hier durch das Ablassorgan 12, das als federbelastetes Ventil ausgebildet ist, erfolgen. Es ist jedoch auch denkbar, dass dem Behälterorgan 4 in regelmäßigen Abständen auf bekannte Weise Inertgas zugeführt wird und im Wesentlichen CO₂-Gas abgelassen wird.

Im vorliegenden Fall weist eine Auswerteanordnung 14 eine CO₂- /O₂-Sensoranordnung 15 auf, die eine Transmitterbox 16 aufweist, in die ein Analysegas über eine Absaugleitung 18 mittels einer Analyselanze 20 angesaugt wird. Die Analyselanze 20 reicht hierbei in den Kaffee 6 hinein. Die Transmitterbox 16 ist im vorliegenden Ausführungsbeispiel kabellos mit einer Steuereinrichtung 22 verbunden. In der Transmitterbox ist ein CO₂-Sensororgan 24 zur Messung des CO₂-Gehaltes, ein O₂-Sensororgan 26 zur Messung des O₂-Sensorgehaltes, eine als Membranpumpe 28 ausgeführte Ansaugpumpe zur Ansaugung des Analysegases sowie ein Interface 30 zur kabellosen Übermittlung der Messergebnisse an die Steuereinrichtung 22 vorgesehen. Die kabellose Verbindung ist hier mit dem Bezugszeichen 32 versehen. Es sollte deutlich sein, dass auch eine konventionelle, kabelgeführte Übertragung möglich ist. Eine CO₂- /O₂-Sensoranordnung 15 besteht also im vorliegenden Ausführungsbeispiel aus den Bauteilen Absaugleitung 18, Analyselanze 20 sowie CO₂-Sensororgan 24. Wie bereits erwähnt, reicht die Analyselanze 20, um ein optimales Messergebnis zu erhalten, im unteren Bereich des Behälterorgans 4 in den Kaffee hinein. Es ist jedoch auch denkbar, das Sensororgan 24 im oberen Bereich des Behälterorgans 4, also in dem Bereich, in dem kein Kaffee gelagert ist, auf geeignete Weise ein Analysegas ansaugen zu lassen.

Um das Messergebnis, insbesondere bei einem bis zu einem bestimmten Druck dichten Behälterorgan 4, wie im vorliegenden Fall, nicht zu verfälschen, ist eine Rückführleitung 34 für das Analysegas vorgesehen, dass das Analysegas von der Transmitterbox 16 mittels der Membranpumpe 28 wieder zurück in das Behälterorgan 4 führt. Bei einer hier nicht dargestellten Ausführungsform eines Behälterorgans 4, bei dem Inertgas zugeführt und CO₂ abgeführt wird, ist naturgemäß ein Inertgas-Zuführorgan vorzusehen. Dieses Inertgas-Zuführorgan kann dann in besonders vorteilhafter Weise auch durch die Analyselanze 20 ausgebildet sein.

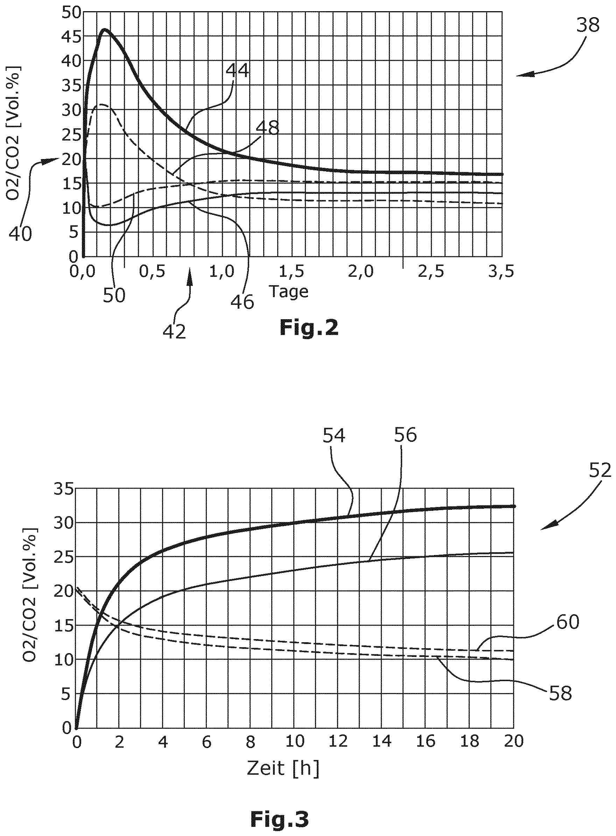

Um nun den optimalen Zeitpunkt zur Beendigung des Ausgasungsprozesses zu bestimmen, ist in der Steuereinrichtung 22 und damit in der Auswerteanordnung 14 ein CO₂- /O₂-Zeitdiagramm 38 (siehe hierzu Figur 2) für den entsprechenden Kaffee hinterlegt, indem ein CO₂-Gehalt-Endwert 40, hier in Höhe von 20 Volumen-%, für einen gewissen Zeitbereich 42 hinterlegt ist. Der Zeitbereich 42 (zwischen 0,3 und 2,3 Tage) wurde hier so ausgewählt, dass der CO₂-Gehalt-Endwert 40 auf jeden Fall beim Abfallen der CO₂-Gehalt-Kurve 44 ermittelt wird. Zudem ist im vorliegenden Diagramm 38 noch eine O₂-Kurve 46 für Filterkaffee dargestellt. Mit den Bezugszeichen 48, 50 sind in dem Diagramm 38 noch die entsprechenden Kurven für Espressokaffee dargestellt. Zu erkennen ist hierbei, dass der CO₂-Gehalt von gemahlenem Espressokaffee nicht so hoch ist, wie der von gemahlenem Filterkaffee, da dieser einen feineren Mahlgrad aufweist. Das heißt beim gemahlenen Espressokaffee ist schon mehr CO₂-Gas beim Mahlprozess entwichen. Demzufolge ist der Sauerstoffgehalt beim gemahlenen Espressokaffee (Kurve 50) auch höher als der beim gemahlenen Filterkaffee (Kurve 46) ohne Inertgasvorgang.

Figur 3 zeigt nun ein Diagramm 52 das den CO₂-/O₂-Gehalt der Lagerungszeit T gegenüberstellt. Da hier das Behälterorgan 4 gasdicht ausgeführt ist und kein Gas abgeführt wird, ist ein Gasabfall nicht festzustellen. Unterschiede ergeben sich hier aufgrund einer anderen Kaffeesorte, eines anderen Röstgrades sowie einem anderen Mahlgrad. Hierbei stellt die Kurve 54 den Verlauf des CO₂- Gehaltes von Columbia-Arabica-Kaffee dar, die Kurve 56 den Verlauf des CO₂-Gehaltes von Vietnam-Robusta-Kaffee dar, die Kurve 58 den O₂-Gehalt von Columbia-Arabica dar und die Kurve 60 den Verlauf von des O₂-Gehaltes von Vietnam-Robusta-Kaffee dar.

Durch diese Gegenüberstellung sollte deutlich sein, wie bspw. variierende Umgebungsbedingungen, wie z.B. Temperaturen, sich auf das Entgasungsverhalten auf die Dauer der Entgasungszeit auswirken. Ebenso können sich auch Änderungen oder Schwankungen am Produkt selbst auf die Zeit auswirken. Dies kann durch die die erfindungsgemäße Anordnung dokumentiert und angepasst werden, so dass eine gleichbleibende Qualität vom Endprodukt gewährleistet ist.

## Patentansprüche

1. Ausgasungsanordnung für frisch gerösteten Kaffee (6) mit einem Behälterorgan (4), in dem der Kaffee (6) gelagert ist, das zumindest eine Einlass- bzw. eine Auslassöffnung (8, 10) aufweist, wobei zumindest eine CO₂-Sensoranordnung (32) mit zumindest einer Ansaugpumpe (28), einem CO₂-Sensororgan (24) und einer Auswerteanordnung (14) für den CO₂-Gehalt eines dem Behälterorgan (4) entnommenen Analysegases zur Überwachung eines Ausgasungsprozesses vorgesehen ist, die über eine Absaugleitung (18) mit dem Behälterorgan (4) fluidisch verbunden ist, wobei in der Auswerteanordnung (14) ein CO₂-Endwert für den CO₂-Gehalt hinterlegt ist, mit dem der jeweilige aktuell gemessene CO₂-Gehalt vergleichbar ist, derart, dass bei Erreichen des CO₂-Endwertes (40) der Ausgasungsprozess zu beenden ist, **dadurch gekennzeichnet, dass** die Sensoranordnung (32) eine Analyselanze (20) aufweist, die in das Behälterorgan (4) hineinreicht und die fluidisch über die Absaugleitung (18) mit einer außerhalb des Behälterorgans (4) angeordneten Transmitterbox (16), die zumindest die Ansaugpumpe (28) und das CO₂-Sensororgan (24) aufweist, verbunden ist, wobei die Transmitterbox (16) kabellos oder kabelgebunden mit einer Steuereinrichtung (22) verbunden ist, wobei die Analyselanze (20) in einem unteren oder mittleren Bereich des Behälterorgans (4) vorgesehen ist, derart, dass die Analyselanze (20) in den Kaffee (6) hineinreicht.

2. Ausgasungsanordnung nach Anspruch 1, **dadurch gekennzeichnet, dass** eine Rückführleitung (34) zur Rückführung des Analysegases von der Transmitterbox (16) in das Behälterorgan (4) vorgesehen ist.

3. Ausgasungsanordnung nach einem der Ansprüche 2 oder 3, **dadurch gekennzeichnet, dass** in der Transmitterbox (16) ein O₂-Sensororgan (26) zur Messung des O₂-Gehaltes vorgesehen ist.

4. Ausgasungsanordnung nach einem der vorhergehenden Ansprüche, **dadurch gekennzeichnet, dass** ein Ablassorgan (12) am Behälterorgan (4) vorgesehen ist.

5. Ausgasungsanordnung nach einem der vorherigen Ansprüche, **dadurch gekennzeichnet, dass** ein Inertgas-Zuführorgan am Behälterorgan (4) vorgesehen ist.

6. Ausgasungsanordnung nach Anspruch 5, **dadurch gekennzeichnet, dass** das Inertgas-Zuführorgan durch die Analyselanze (20) ausgebildet ist.

7. Ausgasungsanordnung nach einem der vorhergehenden Ansprüche, **dadurch gekennzeichnet, dass** in der Auswerteanordnung (14) unterschiedliche CO₂-Zeit-Diagramme für unterschiedliche Kaffeezustände, wie z. B. unterschiedliche Kaffeesorten, Kaffeebohnen, Mahlkaffee, Mahlgrad, etc., hinterlegt sind.

8. Verfahren zum Lagern von frisch geröstetem Kaffee, das heißt in Form von Kaffeebohnen oder Mahlkaffee, in einer Ausgasungsanordnung nach einem der vorhergehenden Ansprüche, **dadurch gekennzeichnet, dass** Kaffee (6) in das Behälterorgan (4) eingefüllt wird, der Ausgasungsprozess mittels der Auswerteanordnung (14) überwacht wird und wobei der Ausgasungsprozess und damit ein Lagerprozess beendet wird, sobald ein vordefinierter CO₂-Endwert (40) in einem vordefinierten Zeitbereich (42) erreicht wird.

## Claims

1. Degassing arrangement for freshly roasted coffee (6) comprising a container component (4) in which the coffee (6) is stored and which has at least one inlet or outlet opening (8, 10), wherein at least one CO₂ sensor arrangement (32) is provided for monitoring an outgassing process, the CO₂ sensor arrangement (32) comprising at least one suction pump (28), a CO₂ sensor component (24) and an evaluation arrangement (14) for the CO₂ content of an analysis gas taken from the container component (4), and the CO₂ sensor arrangement (32) being fluidically connected to the container component (4) via a suction line (18), wherein a CO₂ end value for the CO₂ content is stored in the evaluation arrangement (14), with which CO₂ end value the respective currently measured CO₂ content can be compared, in such a way that the outgassing process is to be terminated when the CO2 end value (40) is reached, **characterized in that** the sensor arrangement (32) comprises an analysis lance (20) which extends into the container component (4) and which is fluidically connected via the suction line (18) to a transmitter box (16) that is arranged outside the container component (4) and that comprises at least the suction pump (28) and the CO2 sensor component (24), wherein the transmitter box (16) is connected wirelessly or by cable to a control device (22), wherein the analysis lance (20) is provided in a lower or middle region of the container component (4) in such a way that the analysis lance (20) extends into the coffee (6).

2. Degassing arrangement according to claim 1, **characterized in that** a return line (34) is provided for returning the analysis gas from the transmitter box (16) to the container component (4).

3. Degassing arrangement according to one of claims 2 or 3, **characterized in that** an O₂ sensor component (26) for measuring the O₂ content is provided in the transmitter box (16).

4. Degassing arrangement according to one of the preceding claims, **characterized in that** a drain component (12) is provided on the container component (4).

5. Degassing arrangement according to one of the previous claims, **characterized in that** an inert gas supply component is provided on the container component (4).

6. Degassing arrangement according to claim 5, **characterized in that** the inert gas supply component is realized by the analysis lance (20).

7. Degassing arrangement according to one of the preceding claims, **characterized in that** different CO₂ time diagrams for different coffee conditions, e.g. different types of coffee, coffee beans, ground coffee, degree of grinding, etc., are stored in the evaluation arrangement (14).

8. Method for storing freshly roasted coffee, i.e. in the form of coffee beans or ground coffee, in a degassing arrangement according to one of the preceding claims, **characterized in that** coffee (6) is filled into the container component (4), the degassing process is monitored using the evaluation arrangement (14) and wherein the degassing process and thus a storage process is terminated when a predefined CO2 end value (40) is reached in a predefined time range (42).

## Revendications

1. Arrangement de dégazage pour café fraîchement torréfié (6) avec un organe de réservoir (4), dans lequel le café (6) est supporté, qui comprend au moins une ouverture d'entrée ou de sortie (8, 10), au moins un ensemble de capteurs de CO2 (32) avec au moins une pompe d'aspiration (28), un organe capteur de CO2 (24) et un dispositif d'évaluation (14) de la concentration en CO2 d'un gaz d'analyse prélevé dans l'organe réservoir (4) pour la surveillance d'un processus de dégazage, qui est relié fluidiquement à l'organe réservoir (4) par une conduite d'aspiration (18), une valeur finale de CO2 pour la concentration en CO2 étant mémorisée dans le dispositif d'évaluation (14), à laquelle la concentration en CO2 actuellement mesurée peut être comparée, de telle sorte que, lorsque la valeur finale de CO2 (40) est atteinte, le processus de dégazage doit être arrêté, **caractérisé en ce que** l'ensemble de capteurs (32) comprend une sonde d'analyse (20) qui pénètre dans l'organe de réservoir (4) et qui est reliée fluidiquement, par l'intermédiaire de la conduite d'aspiration (18), à un boîtier de transmission (16) disposé à l'extérieur de l'organe de réservoir (4), qui comprend au moins la pompe d'aspiration (28) et l'organe capteur de CO2 (24), le boîtier transmetteur (16) étant relié sans câble ou par câble à un dispositif de commande (22), la sonde d'analyse (20) étant prévue dans une zone inférieure ou centrale de l'organe de réservoir (4), de telle sorte que la sonde d'analyse (20) pénètre dans le café (6).

2. Arrangement de dégazage selon la revendication 1, **caractérisé en ce qu'**une conduite de retour (34) est prévue pour le retour du gaz d'analyse de la boîte de transmission (16) dans l'organe de réservoir (4).

3. Arrangement de dégazage selon l'une des revendications 2 ou 3, **caractérisé en ce qu'**un organe de détection d'O2 (26) est prévu dans le boîtier de transmission (16) pour mesurer la concentration en O2.

4. Arrangement de dégazage selon l'une des revendications précédentes, **caractérisé en ce qu'**un organe de drainage (12) est prévu sur l'organe de réservoir (4).

5. Arrangement de dégazage selon l'une des revendications précédentes, **caractérisé en ce qu'**un organe de distribution de gaz inerte est prévu sur l'organe de réservoir (4).

6. Arrangement de dégazage selon la revendication 5, **caractérisé en ce que** l'organe de distribution de gaz inerte est formé par la sonde d'analyse (20).

7. Arrangement de dégazage selon l'une des revendications précédentes, **caractérisé en ce que** différents diagrammes CO2-temps sont enregistrés dans l'arrangement d'analyse (14) pour différents états du café, comme par exemple différents types de café, de grains de café, de café moulu, de degré de mouture, etc.

8. Méthode de stockage de café fraîchement torréfié, c'est-à-dire sous forme de grains de café ou de café moulu, dans un arrangement de dégazage selon l'une des revendications précédentes, **caractérisée en ce que** le café (6) est introduit dans l'arrangement de réservoir (4), le processus de dégazage est surveillé au moyen de l'arrangement d'évaluation (14), et le processus de dégazage, et donc un processus de stockage, étant arrêté dès qu'une valeur finale de CO2 prédéfinie (40) est atteinte dans une période prédéfinie (42).
